# EUROPEAN PATENT APPLICATION

(11) **EP 0 694 309 A2**
(43) Date of publication of application: **31.01.1996**
(21) Application number: 95305294.1
(22) Date of filing: 28.07.1995
(51) Int. Cl.: A61K 39/085, C07K 14/31, C07K 16/12

(54) **Vaccine, antigens and antibodies containing compound for inhibiting and preventing induced staphylococcus infection**

(30) Priority: 29.07.1994 JP 178581/94
(71) Applicant: THE KITASATO INSTITUTE, Minato-ku Tokyo (JP)
(72) Inventor: Takahashi, Takashi, Kita-ku, Tokyo (JP); Sasaki, Takeji, Kohta-ku, Tokyo (JP); Iwai, Yuzuru, Narita-shi, Chiba (JP); Hashimoto, Takashi, Chofu-shi, Tokyo (JP)
(74) Representative: Woods, Geoffrey Corlett

(57) **Abstract**

An antigen comprising in inactivated form the isolated fraction of proteins having a molecular weight of 10,000 to 70,000 derivable from a mixture of cell surface proteins of a bacterium belonging to the genus Staphylococcus and metabolites of a culture medium of said bacterium.

## Description

The present invention relates to a vaccine, antigens and antibodies containing composition for inhibiting and preventing induced Staphylococcus infection. More particularly the present invention concerns a vaccine for preventing Staphylococcus infection, its prevention by antibody, therapeutic antibody and production thereof.

In the past years, inactivated vaccine comprising hemolysin and coagulase extracted from culture medium of Staphylococcus aureus was used for treatment of Staphylococcal infections. However, since newly developed antibiotics had been used for Staphylococcus infections, needs for vaccine for treatment of Staphylococcal infection had been reduced, and finally the vaccine was not used and consigned to lasting oblivion. No development and ideas for the vaccines in this connection have been appeared. The vaccine for Staphylococcus in the past was not specified in its effective component of vaccine, showing with incomplete purification and unclear effective component, accordingly its preventive effect is not clear.

Staphylococcus is a Gram-positive bacterium and exists as a normal inhabitant, i.e. indigenous bacterium, of human skin and mucous membrane. Although most strains produce biologically active toxins and enzymes, little is clarified as yet concerning the pathogenesis of staphylococcal disease which has been markedly aroused in recent years by an apparently increasing incidence of severe illness, particularly in hospital environments where many strains are intensive to the antimicrobial agents which have so successfully cured to mortality of disease cuased by other pyrogenic cocci (Bacterial and Mycotic Infections of man 4th ed, 1991). On the other hand, a typical vaccine of staphylococci is tried, namely an autogenous vaccine. Some doctors have tried this vaccine, however, the efficacy is yet unclear, because the strain has to be isolated from the patient and be inactivated by formalin to prepare the vaccine and the vaccine thus prepared has to be injected for long period. This vaccination may inhibit the secondary infection to human caused by staphylococci.

For animals, there is a vaccine in which whole cell and culture broth are separately inactivated with formalin and mixed finally. This vaccine is to prevent mastitis of animals (Watson D.L., Res. vet. Sci.; 45, 16 - 21, 1988). For mastitis, the other vaccines are highly purified α and β toxin (Infect. Immun., 17, 250 - 256, 1977) and fibronectin-binding protein (FnBP-A)(EUR, 294 - 349, 1988, Vaccine, 12, 11, 918 - 922, 1994). In the idea for vaccine, a subunit vaccine to use of components from surface protein and polysaccharide(T. J. Foster, Vaccine 9, 4, 221 - 227, 1991) has known and synthetic ST enterotoxin comprising 4n to 18n amino acids is said to be useful for inducing in vivo antibodies (U.S. Pat. 4,499,080). But these are not vaccine for staphylococcus infection due to only for use of general vaccine antibody inducing agent.

In our present invention, the strong β hemolytic strain, which is a methicillin resistant strain (MRSA in paticular) isolated from patients, is cultured in anaerocolumbia agar with rabbit blood (BBL Japan) by BBL anaerobic Jar system. This strain produces TSST-1 and strong β hemolysin. After centrifugation of the cultured broth, supernatant is filtered and ammonium sulfate is added to a concentration of about 65 - 85% and the precipitate is preserved for later use. The whole cells are immediately suspended into hypertonic buffer solution and are stirred at the temperature below the denaturing point of protein. After centrifugation, ammonium sulphate is added to the cell extract and the precipitate is mixed with the supernatant precipitate. The mixed solution is treated with an ion exchange column chromatography to eliminate impurities such as medium composition. The fraction having the molecular weight of about 10,000 - 70,000 is obtained by gelfiltration. The main constituents are TSST-1, hemolysin, and enterotoxin, however they contain neither endotoxin nor whole cells. The inactivated vaccine of the present invention is based upon the discovery that the antigens have been obtained by mixing the cell surface extract with supernatant extract. In addition, the strain is the methicillin resistant bacterium isolated from patients showing excellent antigenic activity and also the derived antigen is extracted from cell wall using a hypertonic buffer solution under mild conditions, and moreover the specific antigen includes super antigen and major enterotoxins, and the like. The same enterotoxins, for example A, B and C, are produced not only by Staphylococcus aureus but also by the other species of Staphylococcus. The vaccine derived from the other species contains the same compositions as of Staphylococcus aureus and may be cross-reactive, and the antibody possesses anti-superantigen neutralizing antibody.

The present invention is directed to provide a process for producing an antigen preparation for inhibiting or preventing induced staphylococcal infections in human a vaccine containing such an antigen or antigens, a process for producing antibodies for inhibiting or preventing human and animals infections induced by staphylococcal compositions containing such antibodies. According to a feature of the present invention, there is provided a process for isolating two antigens for preventing or inhibiting human staphylococcal infection comprising separating the whole cells of at least one strain of Staphylococcus from cultured broth, extracting at least some antigens of cell surface and at least few antigens of exotoxin and fractionating the resultant antigen from the extract thus obtained. This vaccine is characterized by containning superantigen (for example TSST-1) and the antibody possessing superantigen neutralizing antibody.

For the purpose of the present invention, both antigens derived from extracts of cell surface and cultured broth are mixed. The mixed solution is treated by ion exchange chromatography and gelfiltration chromatography to obtain the effective fraction. The fraction which has succeeded in obtainning vaccine for human and animals is free from endotoxin and whole cells. The preparation method of this vaccine can be applied to all Gram-positive bacteria, although Staphylococcus aureus is most important. These bacteria may be used solely or in combination. Various mutant strains thereof may also be used for the purpose of the present invention insofar as they are capable of inducing oppotunistic infections. Accordingly, if desired, the vaccine of the present invention can be comprising two more component antigens originated from virulent microoragnisms of other spiecies possessing superantigens. In the examples and experiments described hereinafter, Staphylococcus aureus KT-472 is used. The present strain has been deposited at American Type Culture Collection, on 24th July, 1995 and assigned deposit No. ATCC 55697. When this strain is cultured, for example by using blood agar plate, a strong hemolysin or coagulase is produced.

The biochemical characteristics of the strain
KT-472 are as follows:
- 1) Morphology: : cocci, gram-positive, 0.8 - 1.0µm
- 2) Growth property: : aerobic (facultative anaerobic)
- 3) Growth on various media: :

a) Staphylococcus agar 110 medium [commercial medium ] [pH about 7.2 - 7.4 3 colonies formed are smooth, small, yellow or pale orange color.
b) Trypticase soy agar [commercial medium ] [pH 7.2 - 7.4]
   Colonies are uniform, round and smooth surface
c) Brain heart infusion broth [commercial medium ] [pH 7.2 - 7.4]
   Growing uniformity, in the medium

- 4) Hemolysis on blood agar: : rabbit, sheep, goat and horse
- 5) Coagulase production: : positive, type II
- 6) Enterotoxin production: : A, B and C, TSST-1, Hemolysin
- 7) Growth range: : pH 4.8 - 9.4, temperature 10 - 45°C
- 8) Decomposition of sugar: : positive (+) sucrose, D-mannitol, D-mannose, lactose, D-trehalose, D-galactose, D-fructose
: negative (-)

Arabinose, cellobiose, fucose, xylose, elegitose, salicine, rhafinose
- 9) Miscellaneous: : coagulase (+), motility ( -)
- 10) Drug resistant: : methicillin

Physiological characteristics of antigen are as follows:
- 1) Color and smell: : pale yellow, non specific smell,
- 2) Toxicity: : 10µg protein N/0.1 ml injected intraperitoneally in mice (four weeks age); all death within 3 hours,
- 3) Stability: : denatured at room temperature for ten days, stable at 4 - 8°C for about six months, stable below -20 °C, inactivated at 100°C heating for 60 min.
- 4) Solubility: : Soluble in water or buffer solution,
- 5) pH: : Dried preparations show week acidity,
- 6) Molecular weight: : in 7.5% polyacrylamide gel (SDS PAGE):
electrophoresis ; 10,000 - 70,000,
main component ; 20,000 - 40,000
- 7) Major component: : enterotoxin A, B and C, TSST-1, hemolysin.

To antigen, it is possible, if necessary, to add appropriate adjuvant such as, aluminum phosphate, aluminum hydrooxide, muramyldipeptide and general oil adjuvant to obtain an antigen solution suitable for immunizing mammal and animals. Dosage of vaccine of the resultant solution containing antigen is usually 2 - 50 µg protein N per adult (body weight of about 50 kg), if desired, preferably 2 - 10 µg protein N. The mixture of the surface extract and cultured broth prepared by the process hereinabove is preferablly purified to the fraction of molecular weight 10,000 - 170,000, further is preferablly purified to the fraction of molecular weight 10,000 - 70,000. The purified fraction is inactivated by formalin and the like. Staphylococcal infection can widely be prevented and inhibited by this antigen. The resultant antibodies can also be applied for medical treatment and the composition containing the antigen and antibody is expected to have preventive effect.

Microorganisms, which can be used as a source of cell surface component antigens and culture broth extracts of the present invention, can be cultured in a conventional manner for culturing various strains of corresponding species. Culturing may be effected by using either synthetic or organic media, and liquid media may be preferred for massproduction. For example, the culturing may usually be effected at temperature of 25 - 40°C (preferably at about 36°C) and at pH of 5.0 - 8.5 (preferably at about 7.2) under slight anaerobic condition for 20 - 70 hours. Medium for culturing the microorganism is exemplified as follows.
Medium A : Trypticase soy broth [commercial medium ]
Medium B : Brain heart infusion [commercial medium ]

Medium A and B are preferable for Staphylococcus aureus KT-472, and preferred methods for the preparation of vaccine of the present invention are illustrated as follows.

Microorganisms are cultured at 30 - 37°C, pH 6.5 - 8.5 for 18 - - 48 hours to obtain seed culture. The seed culture is then transferred to main medium comprising the same composition, and cultured for 24 - 72 hours under the same conditions. After completion of culturing, the cells are separated from cultured broth, for example, by centrifugation (8.000 rpm/30 min.) and suspended immediately in a suitable solution well known in the art such as a 0.01M - 0.05M tris-HCl buffer containing 0.5M - 2M NaCl (pH 6.5 - 8.5), 0.01M - 1M phosphate buffer containing 0.5M - 2M NaCl (pH 6.5 - 8.0) and the like.

A suitable non-ionic surfactant, such as triton X-100 (0.01 - 0.001 %w/v)(commercial product of RHOM and Hass. U.S.A.) may, if desired, be added. The cell suspension is treated under stirring for 48 - 112 hours at the temperature below denaturing point of antigenic protein with sonication of, for example, 10 - 20 KHz/2-5 min. to extract the desired surafce antigen. After stirring, sonicated cell suspension is treated, for example, by centrifugation (8,000 rpm/30 min.).

Supernatant is salted out with, for example, 50 - 85% ammonium sulphate and the like to obtain cell surface extract. The supernatant of cultured broth separated from cells is salted out with, for exapmle, 50 - 85% ammonium sulphate and the like to obtain the cultured broth extract. Both extracts from cell surface and cultured broth, containing desired antigen are then mixed, and fractionated and purified in a conventional manner, for example, by column chromatography, isoelectric point precipitation and partition precipitation using cold solvent such as ethanol and the like. If desired, the resultant solution is treated with a suitable inactivating agent such as formalin (0.1 - 0.8 %w/v), followed by dialysis against similar buffer solution to remove formalin.

The solution containing the antigen is diluted with a suitable buffer solution such as 0.01 - 0.1M phosphate buffer saline (pH 6.2 - 7.2) to adjust the protein N concentration to 2 - 50µg protein N/ml. To the dilute solution is added aluminum hydroxide as adjuvant at final concentration of aluminum about 100 - 200 µg/ml to adsorb the antigen. Muramyldipeptide, may, if desired, be used in place of alminum hydroxide. To the mixture is added a suitable antiseptic reagent such as thimerosal (0.05 - 0.01 %w/v) to obtain antigen solution of present invention. In the cell surface extracts, instead of treatment with ultrasonic waves, it is also possible to add ammonium sulphate about 50 - 85% saturation (for example 75%) with stirring to dissolve ammonium sulphate. The supernatant is discarded.

The precipitate is dissolved in a suitable buffer solution such as 0.05 - 0.5M phosphate buffer solution (pH about 6.5 - 8.0) followed by dialysis in the buffer solution at cold temperature. The residual solution is centrifuged (e.g. 8,000 rpm/30 min.) and the resultant extracted solution is fractionated and purified in a similar manner hereinabove described. In order to obviate denaturation of antigen of the present invention, the above mentioned procedure may advantageously be carried out at cold temperatures, for example, below 10°C. The vaccine thus prepared may be preserved over extend period of time. The dosage of vaccine of the present invention may vary, depending upon various factors, for example, types and symptoms of the staphylococcal infection being the target of administration. However, it is usually possible to administer the vaccine to humans at daily dose of, for example, 0.1 to 0.2 ml by subcutaneous or intramuscular injection. The immunization may be effective.

It is also possible, if desired, to administer the vaccine of present invention consecutively. It is possible to inhibit the infection of the wild strains or at least of the corresponding species to human and animals. If desired, it is also possible to immunize a mammal and avian with the vaccine of present invention to produce an immunological antibody, and the administration can be carried out for usual animals in a conventional manner and the resultant antibody is reproducible.

### Test of the vaccine

Each vaccine prepared by the method of example 1 was subjected to staining test, bacterial culturing test and acute abnormal toxicity test, all being effected according to "Minimum requirement of general test for biologics (1994)" issued by the Ministry of Welfare, the Japanese government in the following manner. Nothing usual was noted.
(1) Staining test:
   A sample vaccine (about 10 ml) was put in a test tube and centrifuged (about 2,000g/30 min.) to form precipitate. Then, Gram's strain was applied to the sample for staining test. The sample was observed microscopically (× 1,000). No microorganisms were observed.
(2) Bacterial negative test:
   A sample vaccine (0.2 ml) was divided into two equal portions. Each fraction was cultured for ten days at 30 - 32°C using thioglycolic acid medium (commercial product of Difco, U.S.A.). Nothing usual was noted on the 2nd, 3rd, 7th, 10th and 14th days after starting the culture.
(3) Test for abnormal acute toxicity:
   Five mice of three weeks age were abdominally injected with a sample vaccine (each 0.5 ml). Each animal was observed for subsequent seven days. No usual toxicity was noted.

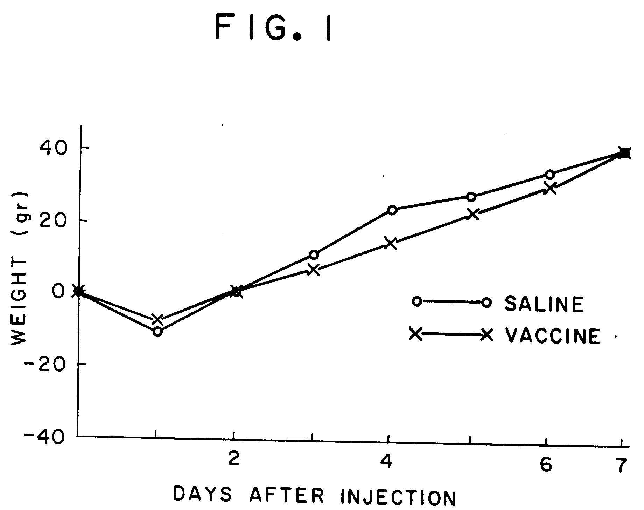

FIG. 1 is test for proving no abnormal toxicity.

### EXAMPLES

In the following non-limiting examples for illustrating the invention, the culturing was effected at a temperature of 30 - 37 °C under slight anaerobic conditions and the test animals, ddY mice (Clean, SPF)(SLC Japan) each group consisting of 10 to 60 animals, or guinea pig (about 200 g), were used unless otherwise specified.

### Example 1 Preparation of vaccine:

Staphylococcus aureus KT-472 (isolated from MRSA patient) was cultured for 24 hours by using anaerocolumbia agar with rabbit red blood (BBL Japan) under BBL culturing Jar system to obtain seed. The seed was transferred to preculture medium A (100 ml) and cultured for 8 hours under the similar conditions hereinabove. The seed culture was transferred to main culture medium consisting of the same composition (10,000 ml) and cultured for 24 hours under similar conditions. Culture broth was centrifuged and separated the broth from cells. Solid ammonium sulphate was added to the cultured supernatant broth at a 85% saturation. Cells were suspended in a 0.01M phosphate buffer containing 1M NaCl (pH 8.0. 100 ml) and stirred for 72 hours at low temperature and cell debris was removed by centrifugation.

Ammonium sulphate was added to the cell removed supernatant at 85% saturation, and stirred at 4°C for further 24 hours. The precipitates of the extracts of supernatant and cells were collected, respectively, by centrifugation (8,000 rpm/30 min.). Then the precipitates were mixed and dissolved in a 0.05M tris-HCl buffer (100 ml, pH 8.0) and were subjected to dialysis against a similar buffer solution, and centrifuged (12,000 rpm/20 min.) to remove impurities. The resultant supernatant (about 120 ml) contained protein N about 2 - 3 mg/ml and was put into visking tube (Union Carbide, U.S.A.) to concentrate the amount to 1/10.

The combined cell extracts and supernatant were eluted by means of ion cxchange column chromatography (Whatman DE-52 60g, U.S.A.) with the solution of 0.05M tris-HCl buffer containing 0.09M NaCl (total elution volume 2,100 ml) for removal of impurities and concentrated the volume to 1/1,000 by the same conditions hereinabove. Eluate was concentrated by visking tube (about 3 ml). The eluate was gel filtrated with sephacryl-200 (Pharmacia, Sweden) eluted with 0.5M NaCl 0.01M phosphate buffer solution (pH 7.2). Fractions were collected by monitoring at 280 nm. The fractions of molecular weight (10,000 - 70,000) were collected. The combined active fractions exhibited hemolytic ability to rabbit red blood cell at a dilution ratio of 1 : 1024. Thus the active principle was inactivated with 0.3% formalin for 10 weeks at 4 °C. On each occasion, the fraction was diluted with 0.01M phosphate buffer saline (pH 7.0) to give a concentration of protein N of 5 to 50 µg/ml. Inactivation was carried out in the same manner as above. After removal of formalin by dialysis against the same buffer solution, aluminum hydroxide gel was added to final concentration of 20 µg/ml to adsorb the antigens. The pH of combined antigen solution was adjusted to 6.5 and thimerosal, an antiseptic agent, was added to the solution.

### Example 2 Inactivation test by intradermally reaction of the rabbit:

About 10µg of protein N of the antigen obtained in Example 1 were injected intracutaneously into rabbit (weighning about 2kg, New Zealand white, Gokita Japan). The injected rabbit died within 15 hours. Intracutaneous injection of 200µg protein N of the inactivated antigen in a rabbit showed no death, accordingly the intradermal reaction of the inactivated antigen was negative.

### Example 3 Cytotoxic effect on Vero cells:

Vero cells were grown in MEM (Gibco, U.S.A.) added with 5% fetal calf serum in 96 holes microplate. Dilution series of an antigen before inactivation or inactivated antigen, each 25 µl, were added thereto. Cells were incubated for 5 days at 37°C, and cell degeneration was observed. Results are shown in Table 1. Inactivated antigen showed no cytotoxic effect on Vero cells.

**Table 1**

| Cytotoxic effect on Vero cells | | | | | |
|---|---|---|---|---|---|
| Antigen/dilution | origin | ×10 | ×100 | ×1,000 | ×10,000 |
| Before inactivation | + | + | + | + | + |
| Inactivated | ± | - | - | - | - |

### Example 4 Lethal dose test of the prepared antigen:

Saline solutions of the antigen before inactivation, containing 10 to 100 µg protein N, prepared in Example 1 were injected intraperitoneally into ddY mice (SPF, four weeks age), 10 mice in each group, and conditions of mice were observed for seven days. All the mice administered with 100 - 50 µg showed death within three hours after injection. The other groups died within twenty hours after injection. Results are shown in Table 2.

**Table 2**

| Lethal dose test of the prepared antigen | | | |
|---|---|---|---|
| protein µg/0.1 Nml mouse) | number of animals | mortality | survivor (%) |
| 100 | 10 | 10 | 0 |
| 75 | 10 | 10 | 0 |
| 50 | 10 | 10 | 0 |
| 20 | 10 | 10 | 0 |
| 10 | 10 | 3 | 70 |

### Example 5 Vilulence test of the strains used in experiments:

Lethal dose tests were conducted using the strains of Staphylococcus aureus, TSST-1 producing strain A and TSST-1 non-producing strain B, isolated from the patients. The strains were cultured on anaerocolumbia agar with rabbit blood agar (BBL Japan) in BBL anaerobic culture Jar system for 24 hours at 37°C. Ten fold dilution series of cell suspension were prepared. Diluted cell suspension of 10¹⁰cfu/ml to 10⁸cfu/ml was injected, 0.1 ml/mouse, intraperitoneally into ddY mice (four weeks age). Lethality was observed during 14 days after injection. Results are shown in Tables 3 and 4. As can be seen, 80% of mice injected intraperitoneally with strain (A) 10⁸cfu/mouse died, and over 50% of mice injected i.p. with strain (B) 10⁸cfu/mouse died.

**Table 3**

| Virulence test for the strains | | | |
|---|---|---|---|
| number of cells | number of mice | number of death | survivor (%) |
| 4 × 10¹⁰cfu | 10 | 10 | 0 |
| 4 × 10⁹ cfu | 10 | 10 | 0 |
| 4 × 10⁸ cfu | 10 | 8 | 20 |

Strain (A)TSST-1 producing strain was used (i.p.injection).

**Table 4**

| number of cells | number of mice | number of death | survivor (%) |
|---|---|---|---|
| 3 × 10¹⁰cfu | 10 | 10 | 0 |
| 3 × 10⁹ cfu | 10 | 10 | 0 |
| 3 × 10⁸ cfu | 10 | 6 | 40 |

Strain (B)(TSST-1 non producing strain) was used (lP injection).

### Example 6 Lethal dose of prepared antigen:

The solution containing 2.5, 5 and 10µg protein N of vaccine obtained in Example 1 was prepared, respectively, and was adsorbed onto alminum hydroxide adjuvant (200 µg/ml). The vaccine 0.2 ml was subcutaneously injected in 120 mice (ddy, SPF, 4 weeks age, SLC, Japan). After three weeks, the mice, 10 mice in each group, were challenged by toxin and viable cells. The protein in the challenged toxin was adjusted to 20 µg/ml, and its 0.1 ml was intraperitoneally injected. Viable cells were diluted with hemacell (Hoechst, Germany) and the solution of 10⁸cfu/ml to 10¹⁰cfu/ml were intraperitoneally injected, and conditions of mice were observed during 14 days. Results are shown in Tables 5, 6 and 7. The results indicated that in the toxin challenged group, 100% of were, survived when immunized with 10µg protein N antigen. In the viable cells of TSST-1 producing strain challenged group, 100% of mice were, survived when immunized with 2.5µg protein N, antigen and in the cells of TSST-1 non-producing strain challenged group, 70% of mice were survived when immunized with 5 µg protein N antigen.

Blood was collected from both of the mice survived after challenging and the mice immuunized without challenging, and was used in the following tests as the antibody and the like.

**Table 5**

| Lethal dose of the prepared antigen: challenged with antigen before inactivation | | | |
|---|---|---|---|
| vaccine (protein N µg/mouse) | number of mice | number of death | survivor (%) |
| 10 | 10 | 0 | 100 |
| 5 | 10 | 2 | 80 |
| 2. 5 | 10 | 4 | 60 |
| control | 10 | 10 | 0 |

Toxin (20 µg/0.1 ml) was injected 1P.

**Table 6**

| Challenged with TSST-1 producing cells | | | |
|---|---|---|---|
| vaccine(protein N µg/mouse) | number of mice | number of death | survivor (%) |
| 10 | 10 | 0 | 100 |
| 5 | 10 | 0 | 100 |
| 2. 5 | 10 | 0 | 100 |
| control | 10 | 9 | 10 |

Viable cells (5 × 10⁸cfu/mice) were injected IP.

**Table 7**

| Challenged with non TSST-1 producing cells | | | |
|---|---|---|---|
| vaccine(protein N µg/mouse) | number of mice | number of death | survivor (%) |
| 10 | 10 | 3 | 70 |
| 5 | 10 | 3 | 70 |
| 2. 5 | 10 | 6 | 40 |
| control | 10 | 9 | 90 |

Viable cells (4 × 10⁹cfu/mice) were injected IP.

### Example 7 Antibody production test (1): Aggulutination antibody titer to viable cells

Suspension of viable cells (TSST-1 producing strain) was prepared with 0.01M phosphate buffer saline containing 0.1 w/v% albumin. Immunized serum (25 ml) was pipetted in the microplate, and series of two fold dilution in microplate with the same buffer hereinabove were prepared. Equal volume of the viable cell suspension was added thereto and mixed. The mixture was incubated at 37°C for 2 hours and then was left at 4°C for overnight, then aggulutination antibody titers were measured. The antibody titer was assayed as 1 : 16 in 10 µg protein N immunized group, and 1 : 8 in 5µg protein N immunized group. Accordingly, though antibody titer is not so high, the antibody titer is increased. Control shows negative titer. Result is shown in Table 8.

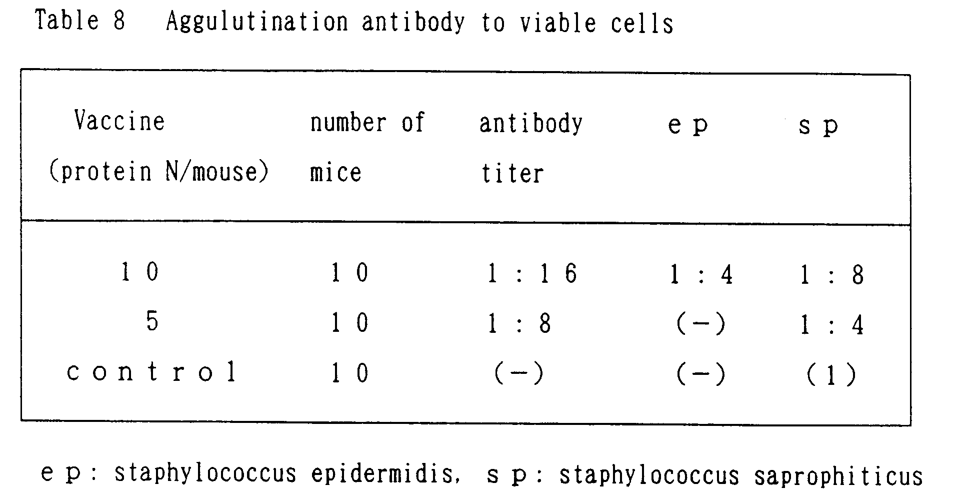

### Example 8 Antibody production test (2): Neutralization to hemolysin

Antigen before inactivation titered to 2¹⁴/ml was diluted to 2³/ml. Immunized serum was prepared to the series of two fold dilution on microplate by using the same buffer solution as described in Example 7. Equal volume of 2³/ml antigen was added to the corresponding serum and mixed. The mixture was allowed to sensitized at 37°C for 2 hours. The equal volume of 2% rabbit red blood cells was added to the corresponding serum and mixed together. Mixture was allowed to react at 37°C for 1 hour. Hemolysin neutralizing antibody titer is shown in 1 : 8 to 1 : 16 in 5µg protein N immunized group and 1 : 8 to 1 : 16 in 10µg protein N immunized group. Hemolysin neutralizing antibody titer in survived mice after challenging is shown in 1 : 64. Result is shown in Table 9.

**Table 9**

| Hemolysin neutralization titer | | |
|---|---|---|
| vaccine (protein N/mouse) | only immunized | survived |
| 10 | 1 : 8∼16 | 1 : 64 |
| 5 | 1 : 8∼16 | 1 : 64 |
| control | (-) | |

### Example 9 Neutralizing titer of immunized serum with vaccine to TSST-1:

Toxin TSST-1 (Toxin Technology, U.S.A., 1 mg/ml) was diluted to adjust to have titered 2³/ml (1 : 8). Immunized serum was prepared to the series of two fold dilution on microplate with buffer as described in the above Example 7. Equal volume of TSST-1 antigen (2³/ml) was added to the corresponding serum and mixed. The mixture was allowed to sensitize at 37 °C for 2 hours and left at room temperature for overnight, then neutralizing antibody was measured by using TST-RPLA [Seiken] [Denka Seiken, Japan ] . The antibody titer was shown in dilution of 1 : 8 in survived mice after challenging with toxin.
Result is shown in Table 10.

**Table 10**

| Neutralizing titer to TSST-1 | | |
|---|---|---|
| weeks after immunized | vaccine(protein N/mouse) | antibody |
| 3 | 10 | 1 : 8 |
| 2 | 10 | 1 : 8 |
| control | saline | (-) |

### Example 10 Infection inhibiting test by passive immunization:

Antibody was prepared by injecting antigen (100 µg protein N) with emulsified Freund imcomplete adjuvant (1 : 1) into rabbit subcutaneously. The antigen (100 µg protein N) was injected into a rabbit at the interval of 2, 4 and 8 weeks, respectively. After 9 weeks, blood was collected from the carotid artery. The immunized serum was diluted serially from 1 : 512 to 1 : 32 with 0.01M phosphate buffer saline. Equal volume of corresponding antigen (hemolysin titer 1 : 128) was mixed and allowed to stand for sensitization at 37°C for 1 hour. Sensitized solutions 0.2 ml each were injected to mice (ddY, 4 weeks age) intraperitoneally. The toxin solution (hemolysin titer 1 : 128/0.1 ml) was injected into mice as control. Each animal was observed for subsequent 7 days. All the mice in control group died within one day and mice in neutralized serum group survived more than 60% in the dilution of serum 1 : 32. The findings hereinabove clearly indicate that the antiserum neutralizes toxin in serum. The possibility of medical treatment was clearly proved. Result is shown in Table 11.

**Table 11**

| Infection inhibition by passive immunization with antiserum | | | |
|---|---|---|---|
| antibody titer | number of mice | number of death | suvivor (%) |
| ×512 | 10 | 0 | 100 |
| 256 | 10 | 0 | 100 |
| 128 | 10 | 0 | 100 |
| 64 | 10 | 4 | 100 |
| 32 | 10 | 4 | 60 |
| control | 10 | 10 | 0 |

### Example 11 Infection inhibition test to viable cells challenged in mice by passive immunization:

Viable cells grown on anaerocolumbia agar with rabbit blood agar (BBL Japan) at 37°C for overnight by BBL anaerobic Jar system, were collected. Viable cells (10¹⁰cfu/ml) were diluted to 10⁹cfu/0.1ml with Hemacell (Hoechst, Germany). Cells were sensitized with corresponding antiserum in the dilution from 1 : 16 to 1 : 256, respectively, and allowed to stand at 37°C for 1 hour. Sensitized cells in the solution were injected to mice intraperitoneally. The cells (10⁹cfu/0.1 ml) were injected intraperitoneally as control. Each animal was observed for subsequent two weeks. All mice in control group died within three days. Animals in the sensitized serum group were survived above 40% in the dilution of the titer in 1 : 16. The efficacy of sensitized serum on the medical treatments was clearly proved. Result is shown in Table 12.

**Table 12**

| Infection inhibiting test by passive immunization with viable cells | | | |
|---|---|---|---|
| antibody titer | number of mice | number of death | survivor (%) |
| ×256 | 10 | 6 | 40 |
| 128 | 10 | 8 | 20 |
| 64 | 10 | 8 | 20 |
| 32 | 10 | 8 | 20 |
| control | 10 | 10 | 0 |

Viable cells (10 LD₅₀cfu/mice) were injected into IP.
Control : death within 3 days

### Example 12 Test for proving no abnormal toxicity:

Antigen (10 µg protein N/ml) 5 ml per guinea pig (about 300g), was injected intraperitoneally in 60 animals to examine abnormal toxicity in accordance with minimum requirement for biological product standard of Japan. The results indicated that animals showed noticeable decrease in body weight as compared with the control. Results are shown in Figure 1.

### Example 13 Endotoxin assay:

Endotoxin was assayed by toxinocolor system II (Seikagaku Kogyo, Japan). The original antigen (not diluted) showed assaying result of 25 pcg. Accordingly, endotoxin of the antigen is below the lower limit of rabbit feber (50 pcg).

## Claims

1. An antigen comprising in inactivated form the isolated fraction of proteins having a molecular weight of 10,000 to 70,000 derivable from a mixture of cell surface proteins of a bacterium belonging to the genus Staphylococcus and metabolites of a culture medium of said bacterium.

2. An antigen according to claim 1 wherein said protein fraction has been inactivated with formalin.

3. An antigen according to claim 1 or 2 derived from a bacterium selected from Staphylococcus aureus, Staphylococcus epidermidis or Staphylococcus saprophyticus.

4. An antigen according to claim 3 derived from methicillin-resistant Staphylococcus aureus (MRSA).

5. An antigen according to claim 4 derived from Staphylococcus aureus KT 472 or a varient or mutant thereof.

6. An antigen according to any one of the preceding claims wherein the metabolites are exotoxins of the bacterium belonging to the genus Staphylococcus.

7. An antigen according to claim 6 wherein the exotoxins are enterotoxin A, B and C, TSST-1 and hemolysin.

8. An antigen according to claim 1 which has the following physiological properties:
(1) colour and smell: pale yellow, non specific smell,
(2) toxicity: 10µg protein N/O.1ml injected intraperitoneally in mice (four weeks age): all dead within 3 hours,
(3) stability: denatured at room temperature in ten days, stable at 4-8°C for about six months, stable below -20°C, inactivated at 100°C heating for 60 min,
(4) solubility: soluble in water or buffer solution,
(5) pH: dried preparations showed weak acidity,
(6) molecular weight: in 7.5% polyacrylamide gel (SDS PAGE):
electrophoresis: 10,000 - 70,000,
main component: 20,000 - 40,000,
and
(7) major component: enterotoxin A, B and C, TSST-1, hemolysin.

9. A vaccine for preventing or inhibiting Staphylococcal infection which comprises an antigen as defined in any one of the preceding claims.

10. A vaccine according to claim 9 which additionally comprises an adjuvant.

11. A vaccine according to claim 10 wherein said adjuvant is an aluminium salt, muramyldipeptide or oil adjuvant.

12. A process for preparing an antigen which comprises culturing a bacterium belonging to the genus Staphylococcus, separating the cultured broth into cells and culture supernatant liquid medium, extracting cell surface protein from the cells, mixing the extracted protein with the supernatant liquid and isolating an antigen protein fraction of molecular weight 10,000 to 70,000.

13. A process according to claim 12 wherein the isolated protein is subjected to an inactivation procedure.

14. A process according to claim 12 or 13 wherein the bacterium is Staphylococcus aureus, Staphylococcus epidermidis or Staphylococcus saprophyticus.

15. A process according to claim 14 wherein the bacterium is Staphylococcus aureus MRSA.

16. A process according to claim 15 wherein the bacterium is Staphylococcus aureus KT 472 or a varient or mutant thereof.

17. A process according to any one of claims 12 to 16 wherein the antigen produced is a mixture of proteins having the following properties:
(1) colour and smell: pale yellow, non specific smell,
(2) toxicity: 10µg protein N/O.1ml injected intraperitoneally in mice (four weeks age): all dead within 3 hours,
(3) stability: denatured at room temperature in ten days, stable at 4-8°C for about six months, stable below -20°C, inactivated at 100°C heating for 60 min,
(4) solubility: soluble in water or buffer solution,
(5) pH: dried preparations showed weak acidity,
(6) molecular weight: in 7.5% polyacrylamide gel (SDS PAGE):
electrophoresis: 10,000 - 70,000,
main component: 20,000 - 40,000,
and
(7) major component: enterotoxin A, B and C, TSST-1, haemolysin.

18. An antibody produced by the antigen as defined in any one of claims 1 to 8.

19. An antibody according to claim 18 for use in the treatment of staphylococcal infection.

20. Use of an antigen as defined in any one of claims 1 to 8 as an immunogen for antibody production.

21. A process for preparing an antigen which comprises dispersing cells of a bacterium belonging to the genus Staphylococcus in a hypertonic buffer solution containing sodium chloride and isolating the desired antigen from the surface of said cells at a temperature which is sufficiently low to obviate denaturing said antigen.

22. A process according to claim 21 in which the bacterium belonging to the genus Staphylococcus is Staphylococcus aureus, Staphylococcus epidermidis or Staphylococcus saprophyticus.

23. A process according to claim 22 in which the bacterium is methicillin resistant Staphylococcus aureus (MRSA).
